(19)

**Europäisches Patentamt**
**European Patent Office**
**Office européen des brevets**

(11) **EP 4 053 206 A1**

(12) # EUROPEAN PATENT APPLICATION

(43) Date of publication:
**07.09.2022 Bulletin 2022/36**

(21) Application number: **21160301.4**

(22) Date of filing: **02.03.2021**

(51) International Patent Classification (IPC):
**C08L 1/02** (2006.01)   **C08K 3/22** (2006.01)
**C08K 3/105** (2018.01)   **C08K 3/11** (2018.01)

(52) Cooperative Patent Classification (CPC):
**C08L 1/02; C08K 3/105; C08K 3/11; C08K 3/22**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fruitful Innovations BV**
**3871 KM  Hoevelaken (NL)**

(72) Inventors:
• **O'CONNOR, Paul**
  **3871 KM Hoevelaken (NL)**

• **BABICH, Igor**
  **7545 GM Enschede (NL)**
• **O'CONNOR, Kimberley**
  **5254 JV Haarsteeg (NL)**
• **BAUTE, Bjorn**
  **5254 JV Haarsteeg (NL)**

(74) Representative: **Hoyng Rokh Monegier B.V.**
**Rembrandt Tower, 30th Floor**
**Amstelplein 1**
**1096 HA Amsterdam (NL)**

(54) **A METHOD FOR THE PREPARATION OF A HYBRID NANO-STRUCTURED COMPOSITE COMPRISING CELLULOSE NANO-PARTICLES AND METAL COMPOUND NANO-PARTICLES**

(57)    The invention relates to a method for the preparation of a hybrid nano-structured composite comprising cellulose nano-particles and metal compound nano-particles, to the nano structured-composite product obtainable by the process and to uses thereof. The method comprises the steps of contacting virgin cellulose with a molten metal salt solvent Mi-S and dissoluting the virgin cellulose, optionally exchanging at least part of metal ions $M_1$ with metal ions $M_2$ and converting at least part of the metal ions $M_1$ and/or optional $M_2$ to metal compound nano-particles, precipitating the cellulose nano-particles and isolating the co-precipitated cellulose- and metal compound nano-particles.

Fig 1.  Example A

EP 4 053 206 A1

## Description

BACKGROUND OF THE INVENTION

### 1. Field of the Invention

This invention relates to a method for the preparation of a hybrid nano-structured composite comprising cellulose nano-particles and metal compound nano-particles, to the nano structured-composite product obtainable by the process and uses thereof.

### 2. Description of the Related Art

The production of cellulose nano-particles is known in the art. For example, Delgado-Aguilar et al in BioResources 10(3), 5345-5355 (2015) describe several processes for preparation of cellulose nanofibers from cellulose containing feedstock involving a combination of chemical pretreatment with high energy mechanical treatment. These processes use costly and non-environmentally chemicals and/or solvents, high energy consumption and complexity in several processing steps.

A known simple route to produce cellulose nano-particles uses acidic solvents ($H_2SO_4$, HCl and or $SO_2$ in water or in organic solvents). However, the yield and the quality of the obtained cellulose nano-particles product is poor because of degradation of the cellulose into sugars and other compounds which will also reduce the applicability of the obtained product.

In WO2017/055407 it is described to dissolve cellulose in a substantially proton-free inorganic molten salt solvent medium, preferably a $ZnCl_2$ hydrate, and precipitating the cellulose with an antisolvent to high aspect ratio nano-cellulose fibrils. However, the properties of the obtained cellulose nanocrystals are not as good as desired in terms of purity, crystallinity, chemical stability and mechanical properties of the product.

In WO2020212616 a further improved process is described for the preparation of micro- or nano crystalline cellulose from virgin cellulose comprising the contacting with a first solvent comprising 40 to 65 wt.% $ZnCl_2$ in water whereby the amorphous cellulose phase is preferentially dissolved over the crystalline cellulose phase producing cellulose micro-particles having an XRD type I structure in a high yield and with high crystallinity and then preferably contacting the obtained product with a second solvent comprising a higher concentration than the first solvent of between 65 and 90 wt.% $ZnCl_2$ in water to produce cellulose nano-particles having an XRD type II structure in a high yield, high crystallinity and high purity.

The production of nano-particles of a metal compound is also well known. However, the problem is to form a nano-structured composite wherein both the nano-particles of the metal compound and cellulose are mixed homogeneously on nano-scale. Nanoparticles typically have a size from about 1 to below 100 nm, preferably below 60 nm or 40 nm and most preferably even below 20 nm. For cellulose nanocrystals having a large aspect ratio this is the size range for the smaller thickness dimension, not the length dimension. A nano-structured composite is a composite where the nano-particles are homogeneously dispersed on nano-scale so most cellulose nano-particles neighbor metal compound nano-particles within the abovementioned nano-scale dimensions, which can be established by Scanning electron microscopy (SEM).

The direct mixing of the metal nano-particles with the nano-cellulose particles is not a complex process but may also cause damage and/or degradation of the cellulose nano-particles, costs a lot of mechanical energy, but mostly the problem is that the products are not homogeneously mixed on nanoscale.

WO2019229030A1 describes a process for the preparation of hybrid inorganic-organic materials, but this process does not result in a nano-structured composite material comprising nano-particles of metal compound and cellulose. This process comprises forming a dissolution of cellulose in an ionic liquid solvent and dispersing an inorganic material, for example alumina or silica, in the cellulose solution adding an anti-solvent to precipitate the nano-cellulose together with the dispersed inorganic material. The resulting co-precipitated inorganic particles are, as opposed to the inorganic particles of the invention, not nano-particles and the hybrid composite material is not a nano-structured composite. The alumina ($Al_2O_3$), dispersed in $ZnCl_2$ together with NC-$ZnCl_2$, results in alumina particles having an average size of 100 nm or more which are not nano-particles.

Farooq e.a. in the International Journal of Biological Macromolecules 154, (2020) 1050-1073, describes hybrid composites comprising cellulose nano-particles modified with metal nano-particles, in particular Zinc-oxide nanoparticles. Farooq describes in the review article several preparation processes to produce cellulose nano-particles involving alkalineacid treatment of cellulose biomass or enzymatic pretreatment or pretreatment with ionic liquids followed by hydrolysis and high-pressure homogenization or mechanical treatment to produce cellulosic nano-particles. Farooq also describes various processes for the preparation of metallic nano-particles wherein different morphologies are obtained in different known ways. It is described to produce Zinc-oxide nano-particles for example by a non-hydrolytic (solution) process using zinc acetate dehydrate. Farooq then also describes a variety of different ways to prepare the hybrid composite material including simple mixing of the cellulose nano-particles in aqueous suspension with metal oxide nano-particles without using any external reducing agent, mixing cellulose nano-particles with metal nano-particles together with reducing agent or surface modifying of the cellulose nano-particles and adding metal-oxide nanoparticles. The preparation processes all start from an aqueous suspension of cellulose nano-particles.

A disadvantage of the described processes for the production of the hybrid composite is that they are complex, involving several process steps and consuming expensive chemicals and therefore are economically and environmentally less attractive.

It is therefore an object of the invention to provide a process for the preparation of a hybrid composite comprising metal nano-particles and cellulose nano-particles that is less complicated and results in a very homogeneous hybrid nano-structured composite material wherein metal nano-particles and cellulose nano-particles are homogeneously mixed on nano-scale. The process of the invention preferably has at least one of the advantages of fewer process steps, less expensive chemicals, lower processing cost, lower energy consumption and lower environmental impact.

BRIEF SUMMARY OF THE INVENTION

According to the invention at least one of the mentioned disadvantages have been overcome by providing process for preparing a hybrid nano-structured composite material comprising cellulose nano-particles and metal compound nano-particles comprising the steps of:

a) Contacting virgin cellulose with a molten metal salt solvent $M_1$-S comprising metal ions $M_1$ and dissoluting the virgin cellulose,

b) Adding a precipitation reactant B to convert at least part of the metal ions $M_1$ of the molten metal salt solvent $M_1$-S to metal compound nano-particles $M_1$-X or exchanging at least part of metal ions $M_1$ with metal ions $M_2$ by contacting with a solution of a salt comprising metal ions $M_2$ and precipitating metal compound $M_2$-X nano-particles directly or by adding a precipitation reactant B,

c) Adding an anti-solvent and precipitating the cellulose nano-particles before, during or after step b),

d) Isolating the co-precipitated cellulose- and metal compound nano-particles to obtain the hybrid nano-structured composite material,

e) Optionally converting the metal compound in the hybrid nano-structured composite material into another metal compound.

In another aspect the invention relates to the hybrid nano-structured composite material comprising metal nano-particles and cellulose nano-particles obtainable by the process of the invention having improved homogeneity and improved properties in use.

Yet another aspect of the invention relates to the use of the hybrid nano-structured composite material in food packaging, biopharmaceuticals, biomedical, cosmetics and electronics applications, for example in solar cells, flexible displays, ultracapacitors etc. where nanoparticles of metal compounds comprising metals like Li, Mn, Ti, Zn, Ru and others are combined with nano-cellulose paper, foils, sheets, tapes etc.

In yet another aspect of the invention relates to the use metal nano-particles or the use of cellulose nano-particles or most preferably the use of the hybrid nano-structured composite material of the invention as a white base pigment, for example but not limited to paint, coatings, adhesives, sealants, inks, plastics, but also in cosmetics such as toothpaste or in food products. The mentioned use relates in particular to replace Titanium compounds such as Titanium dioxide ($TiO_2$) as white pigment in the mentioned applications. It is known that nano-cellulose is white and also that ZnO itself is white, but it was surprisingly found that the hybrid nano-structured composite material of the invention is even whiter than the constituting nano-particles themselves, probably because ZnO is so well dispersed in the nano-cellulose.

BRIEF DESCRIPTION OF THE DRAWINGS

The features and advantages of the invention will be appreciated upon reference to the following drawings, in which:

FIG. 1 Example A is a schematic view of one embodiment of the process of the invention showing general process steps in the production of hybrid composite of cellulose nanocrystals and metal compound nano-particles $NC_s$ - $M_1OH$.

FIG. 2 Example A1 is a specific embodiment of the process of example A showing the use of $ZnCl_2.4H_2O$ as the molten salt solvent to produce $NC_s$ -$Zn(OH)_2$ hybrid composite.

FIG. 3 Example B is a general schematic view of another embodiment of the process of the invention to produce a $NC_s$ - $M_1HCO_3$ hybrid composite.

FIG. 4 Example B1 is a specific embodiment of the process Example B to produce $NC_s$ - $Zn(HCO_3)_2$ hybrid composite.

FIG. 5 Example C is a general schematic view of another embodiment of the process of the invention to produce $NC_s$ - $M_2$ -Acetate hybrid composite.

FIG. 6 Example C1 is a specific schematic view of one embodiment of the process of the invention of example C to produce $NC_s$ - Li-Acetate hybrid composite.

DETAILED DESCRIPTION OF CERTAIN EMBODIMENTS OF THE INVENTION

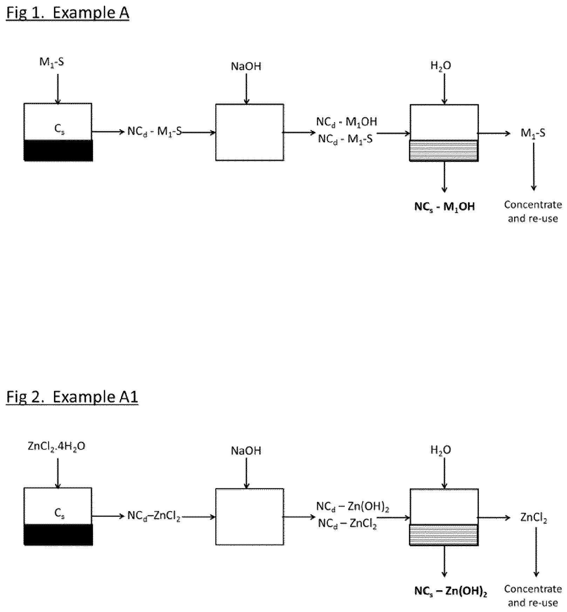

The following is a description of certain embodiments of the invention, given by way of example only and with reference to the drawings. Referring to Example A in FIG. 1, an embodiment of the process of the invention is shown wherein solid virgin cellulose (or virgin cellulose containing feed) $C_s$ is contacted with a molten metal salt solvent $M_1$-S ($M_1$ being the metal). FIG. 2 Example A1, shows a preferred embodiment wherein the molten metal salt solvent $M_1$-S is $ZnCl_2.4H_2O$ ($M_1$ is Zinc). In this first step the solid virgin cellulose $C_s$ is dissoluted to form a suspension or solution of dissoluted nano-cellulose in the molten metal salt ($NC_d$-$M_1$-S preferably $NC_d$-$ZnCl_2$). It is noted that the obtained product from the dissolution step comprises cellulose in nanocrystalline form and may also comprise cellulose polymer, oligomer or monomer that is molecularly dissolved, so the terms suspension and solution are used interchangeably for the product obtained in the dissolution process.

The cellulose particles obtained in this first step can be cellulose particles having XRD type I structure or XRD type II structure. However, it is preferred that the cellulose particles obtained are delaminated cellulose nano-particles having XRD type II structure before the metal precipitation reactant is added as this results in nano-cellulose with a higher aspect ratio and a hybrid composite wherein the cellulose - and metal compound nano-particles are more homogeneously mixed on nano-scale.

In a preferred embodiment, the cellulose particles obtained in a first step are cellulose particles having XRD type I structure which are converted in a separate second step to cellulose nano-particles having XRD type II structure. This second cellulose dissolution step is preferably before addition of the metal precipitation reactant as explained above.

Then a precipitation reactant is added to the obtained cellulose solution $NC_d$-$M_1$-S to convert at least part of the molten metal salt solvent $M_1$-S to an insoluble metal compound $M_1$-X that precipitates as nano-particles in the molten metal salt. In the embodiment shown in FIG. 1 and 2 the metal precipitation reactant is NaOH and the metal nano-particles formed are metal hydroxide $M_1$-OH nanoparticles. In the preferred embodiment of Example A1 in Fig 2. the obtained solution comprises dissoluted nano cellulose $NC_d$ and $Zn(OH)_2$ nano-particles in the $ZnCl_2.4H_2O$ molten metal salt.

The addition of the metal precipitation reactant may also reduce the solvent quality of the molten salt solvent. It was observed that on addition of the precipitation reactant the cellulose nano-particles $NC_d$ start to form a cloudy gel with the precipitated metal compound precipitate $M_1$-OH ($Zn(OH)_2$ in Example A1) and only after adding anti-solvent a clear phase separation occurs between the $NC_d$- $M_1$-OH co-gel and the molten salt solvent.

In a next step an anti-solvent C ($H_2O$ in Example A and A1) is added reducing the solvent power of the molten metal salt and precipitating the cellulose nano-particles $NC_s$ together with the $M_1$-OH nano-particles (preferably. The precipitate is then separated to obtain the solid hybrid composite product $NC_s$-$Zn(OH)_2$. This separation step may involve filtering and/or centrifuging and may comprise one or more washing and/or drying steps.

Preferably the process also comprises a recycling step to regenerate the molten metal salt solvent $M_1$-S, which involves a step of concentrating the diluted molten salt for re-use in the first step. The anti-solvent preferably is water as it allows regeneration of the metal salt solvent $M_1$-S from the separated diluted solvent simply by evaporation of water to the concentration of the molten salt desired in the cellulose dissolution step.

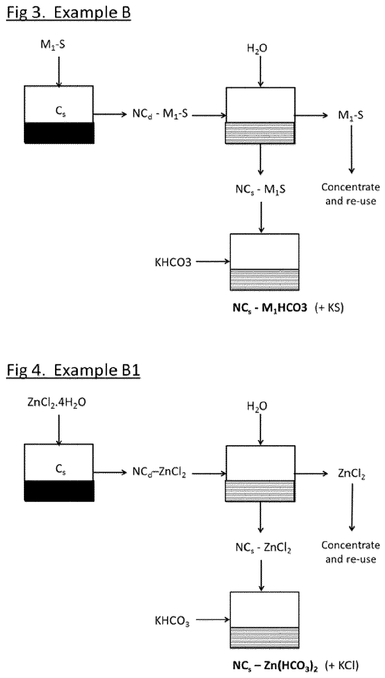

Examples A and A1 in Fig 1 and 2 illustrate the embodiment wherein the metal precipitation reactant is added before adding the anti-solvent to precipitate the nano-cellulose. Examples B and B1 in Fig 3 and 4, show an alternative embodiment of the process of the invention wherein the anti-solvent is added to precipitate the nano-cellulose before adding the metal precipitation reactant.

Herein first a solid virgin cellulose (containing feed) $C_s$ is contacted with a molten metal salt solvent $M_1$-S to produce a solution of cellulose nano-particles, preferably of XRD type II, in the molten metal salt $NC_d$ - $M_1$-S in the same way as described above for Example A. Then anti-solvent C, preferably water, is added to produce a precipitate nano-cellulose $NC_s$ having a size typically below 200 nm to which metals $M_1$-S (preferably $ZnCl_2$) are attached in molecular form.

In a next step, a metal precipitation reactant is added to convert the molten metal salt solvent $M_1$-S to a metal compound $M_1$-X precipitating as nano-particles onto the precipitated nano-cellulose particles forming a very homogeneous hybrid composite of the metal compound and cellulose nanoparticles. In Example B and B1 the metal precipitation reactant is $KHCO_3$ and the hybrid composite comprises cellulose nano-particles $NC_s$ and nano-particles of the modified metal compound $M_1HCO_3$; preferably a hybrid composite $NC_s$ - $Zn(HCO_3)_2$ as shown in Example B1.

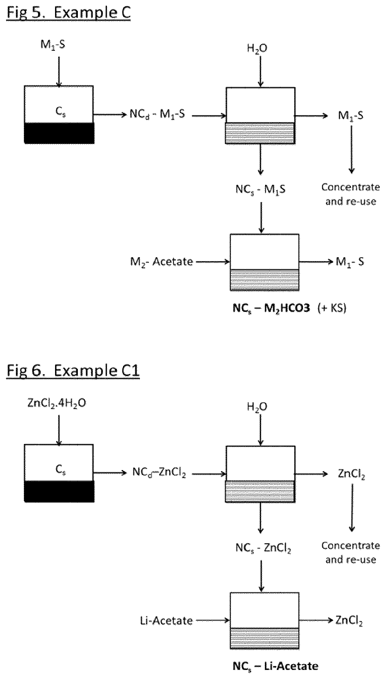

Examples C and C1 in FIG. 5 and 6 show a variant of Examples B and B1 wherein the metal precipitation reactant comprises a metal cation $M_2$ different from the metal $M_1$ in the molten metal solvent. In this embodiment the metal $M_1$ of the molten metal solvent is at least partially exchanged with metal cation $M_2$. Thereafter, the nano-particles of metal $M_2$ compound are formed on the precipitated cellulose nano-particles $NC_s$. In Example C the metal precipitation reactant is $M_2$-acetate and the hybrid composite formed comprises $M_2$-acetate and cellulose nano-particles $NC_s$. Example C1 shows a specific embodiment of the process using Li-acetate precipitation reactant to form a hybrid composite comprising Li-acetate and cellulose nanoparticles. This hybrid composite is useful for instance in solar cells. When the precipitation reactant water is added to the $ZnCl_2.nH_2O$ molten salt cellulose solution, the Cellulose precipitates with a lot of $ZnCl_2$ still present in the precipitate. Then the precipitate is washed with water to remove or reduce the amount of $ZnCl_2$ (what we normally do) or is washed with another metal salt $M_2$-X to replace $ZnCl_2$.

DETAILED DESCRIPTION OF THE INVENTION

The invention relates to a method for the preparation of a hybrid nano-structured composite comprising cellulose nano-particles and metal compound nano-particles, to the nano structured-composite product obtainable by the process and to uses thereof. The method comprises the steps of contacting virgin cellulose with a molten metal salt solvent $M_1$-S and dissoluting the virgin cellulose, optionally exchanging at least part of metal ions $M_1$ with metal ions $M_2$ and converting at least part of the metal ions $M_1$ and/or optional $M_2$ to metal compound nano-particles, precipitating the cellulose nano-particles and isolating the co-precipitated cellulose- and metal compound nano-particles.

The metal compound can be precipitated before or after precipitation of the cellulose nanoparticles. In a preferred embodiment the metal compound particles are precipitated after precipitation of the cellulose nano-particles. When the cellulose nano-particles are precipitated a sort of gel-like precipitate is formed in which the amount of metal ions $M_1$ of the molten salt metal compound is high. Preferably the amount of $M_1$ metal in the precipitate is reduced, preferably by filtering and washing, to an amount needed in view of the desired amount of metal compound nanoparticles on the cellulose nanoparticles followed by precipitating the metal compound nanoparticles $M_1$-X on the nanocellulose. If in view of the envisaged use of the hybrid composite a different metal $M_2$ is desired in the metal compound, a metal $M_2$ ion can be added to the molten salt and precipitating together with or instead of the metal $M_1$ compound. Preferably however, the metal ion $M_2$ is not present in the molten salt and the cellulose is precipitated first, followed by separating the molten $M_1$ metal salt solvent from the precipitated nano-cellulose for recycling and re-use in the first step and then contacting the precipitated nano-cellulose, which still is wetted with the molten metal $M_1$ salt solvent, with a metal $M_2$ salt in one or more steps to exchange the metal $M_1$ of the molten salt solvent with metal $M_2$ followed by precipitating the metal $M_2$ compound nano-particles, optionally washing followed by drying.

The method according to the invention comprises as a first step a) the contacting virgin cellulose with a molten metal salt solvent $M_1$-S comprising metal ions $M_1$ and dissoluting the virgin cellulose.

The molten metal salt preferably is a $ZnCl_2$ hydrate, preferably $ZnCl_2.4H_2O$. The advantage of molten metal salt $ZnCl_2$ hydrate, preferably $ZnCl_2.4H_2O$ is that cellulose is well dissolved with little degradation and depolymerisation of the cellulose is reduced, in particular if the molten salt is also proton-free and preferably comprises a proton scavenger.

In one embodiment the molten metal $M_1$ salt solvent comprises a metal cation $M_2$ other than the metal $M_1$ of the molten salt solvent, preferably comprising a $ZnCl_2$ molten metal salt solvent and a $M_2$ metal chloride, wherein the metal cation $M_2$ preferably is one or more chosen from the group of Li, Mn, Ti, Zn, Nd, Cd, Ag and Ru, most preferably $TiCl_2$, $MnCl_2$, $LiCl_2$ and wherein preferably the amount of $M_2$ metal is less than 20%, preferably less than 10 or 5 mole % of the amount of $M_1$.

The cellulose XRD type II nano-particles nano-particles can be prepared in a process as described in WO2017/055407. However, a preferred process to prepare cellulose XRD type II nano-particles is described in WO2020212616. In this preferred embodiment the method of the invention comprises in step a) contacting virgin cellulose with a first solvent, characterized in that the first solvent is an aqueous solution comprising 40 - 65 wt.% $ZnCl_2$ in water, whereby the amorphous cellulose phase is preferentially dissolved over the crystalline cellulose phase, optionally separating the obtained crystalline cellulose having an XRD type I structure, and contacting the optionally separated crystalline cellulose having an XRD type I structure with a second solvent comprising a higher concentration than the first solvent of between 65 and 90 wt.% $ZnCl_2$ in water to produce delaminated cellulose having an XRD type II structure, wherein the second solvent and preferably also the first solvent is free of proton acid and preferably comprise a proton scavenger. In this process crystalline cellulose nano-particles having an XRD type II structure can be obtained in a high yield, high crystallinity and high purity (i.e. low amount of saccharide oligomers or -monomers and degradation products thereof).

The method according to the invention comprises as step b) adding a precipitation reactant B to convert at least part of the metal ions $M_1$ of the molten metal salt solvent $M_1$-S to metal compound nano-particles $M_1$-X or exchanging at least part of metal ions $M_1$ with metal ions $M_2$ by contacting with a solution of a salt comprising metal ions $M_2$ and precipitating metal compound $M_2$-X nano-particles directly or by adding a precipitation reactant B.

The precipitation reactant B preferably is a base anion X, preferably comprising a hydroxy, carbonate or carboxylate, forming a nano-particle precipitate $M_1$-X with metal $M_1$ and/or $M_2$-X with metal $M_2$. The precipitation reactant B is preferably added as a salt comprising the base anion and hydrogen or a group IA metal cation, preferably sodium or potassium. The precipitation reactant B is chosen from the group of NaOH, KOH, $KHCO_3$, a formiate or acetate salt. The metal compound nano-particles formed are for example nano-particles of $M_1$-OH, $M_1$-$HCO_3$, $M_1$-HCOO⁻ or $M_1$- $CH_3COO$

In another embodiment the precipitation reactant B comprises a metal ion $M_2$ other than metal $M_1$ or the metal $M_1$ is exchanged with metal $M_2$ to form a precipitate of metal $M_2$-X, wherein the metal cation $M_2$ is preferably one or more chosen from the group of Li, Mn, Ti, Nd, Cd, Ag and Ru.

The method according to the invention comprises as step c) adding an anti-solvent and precipitating the cellulose nano-particles before, during or after step b). Preferably, the antisolvent C is water, a ketone or alcohol, preferably water added in an amount to reduce the salt concentration of the molten salt, preferably the $ZnCl_2$ concentration, to between 10 and 30 wt.%, preferably between 15 and 25 wt.%. In step d) the obtained co-precipitated cellulose- and metal compound nano-particles are isolated, preferably by filtration and/or centrifugation, optionally washed and dried to obtain the hybrid nano-structured composite material.

It is noted that WO2019229030A1 describes coprecipitation of cellulose with predispersed inorganic particles. This is different from the process of the invention wherein the metal of the molten metal salt is converted to form nano-particles on the nano-cellulose crystals which, as opposed to the prior art, achieves inorganic compound particles having significantly lower size of below 70, preferably below 50, more preferably below 30 and most preferably even below 20 nm. Another drawback of WO2019229030A1 is that it will only work with certain metal-oxides that allow to be dispersed in the molten salt solvent $ZnCl_2$. This is for instance not possible for oxides of 'heavier' metals like ZnO, NdO, Rare-Earth oxides etc.

In one embodiment of the method of the invention in step b) precipitation reactant B is added to convert at least part of the metal ions $M_1$ to a nano-particles of metal compound $M_{1-}$ X followed by adding anti-solvent C in step c) to precipitate the cellulose nano-particles in the presence of the nano-particles of metal compound $M_1$X, optionally followed by one or more steps of filtration, washing and drying.

In one embodiment of the method of the invention step a) is followed by step c) and then by step b) comprising adding anti-solvent C to the cellulose solution obtained in step a) forming a gel of precipitated cellulose nano-particles, optionally followed by separating the gel and washing to reduce the concentration of the metal salt of the molten salt solvent, followed by addition of precipitation reactant B in step b) to convert at least part of the metal ions $M_1$ to a nano-particles of metal compound $M_{1-}$ X in the presence of the cellulose nano-particles.

In a specific embodiment of this method, the molten metal salt solvent preferably is $ZnCl_2.4H_2O$ and the precipitation reactant B is a hydroxide base, preferably added as sodium hydroxide, resulting in a hybrid composite of cellulose- and Zinc-hydroxide nanoparticles. Optionally the obtained hybrid composite is then treated to convert Zinc-hydroxide nanoparticles to Zinc-oxide nano-particles, preferably by drying at elevated temperatures between 70 and 350°C, preferably between 80 and 300°C, even more preferably between 80 and 280°C.

In an alternative embodiment of the method of the invention step a) is followed by step c) wherein anti-solvent C is adding to the cellulose solution obtained in step a) forming a gel of precipitated cellulose nano-particles, optionally followed by separating the gel and washing to reduce the concentration of the metal salt of the molten salt solvent, followed by step b) wherein at least part of metal ions $M_1$ are exchanged with metal ions $M_2$ by contacting the solution with a solution of a salt comprising metal ions $M_2$, together with or followed addition of precipitation reactant B, forming precipitate of metal compound nano-particles $M_2$X.

The precipitation reactant B and the antisolvent C can be added separately as described above but can also be added together in a single combined step c) and d). Alternatively, a single compound is added that acts both as precipitation reactant B and as antisolvent C to simultaneously precipitate both the metal compound nano-particles and the cellulose nanoparticles. For example, the precipitation reactant B converts the metal $M_1$ of the molten salt into a precipitate and thereby influences the solubility of the dissoluted cellulose to such extent that the cellulose precipitates. If precipitation reactant B is added as a solution of a salt, for example NaOH, in water, both the metal hydroxide compound and cellulose can precipitate.

The method according to the invention optionally comprises as step e) converting the metal compound in the hybrid nano-structured composite material into another metal compound. This can be done for example by thermal decomposition, ion-exchange, reduction or oxidation. Preferably, the metal compound in the hybrid nano-structured composite comprises a hydroxide, carbonate or carboxylate or combinations thereof, preferably carbonate hydroxide compounds, which are optionally converted to metal oxide by thermal decomposition, preferably under vacuum.

Optionally interlinking agents are added in the method to link with the cellulose nanoparticles, which are preferably chosen from the groups of glycerol, citric acid, acetate or chitosan and preferably are organometal compounds of exchange metal $M_2$, preferably $M_2$ - acetate or -citrate and which interlinking agents are preferably added in step b) or c).

The invention also relates to hybrid nano-structured composite comprising cellulose nano-particles and metal compound nano-particles obtainable by the process according to the invention, preferably comprising 2-20 wt% metal compound relative to the total dry weight of the nano-structured composite. The hybrid nano-structured composite preferably comprises cellulose nano-particles having X-Ray Diffraction (XRD) type II structure that preferably have an aspect ratio AR of at least 5, preferably at least 10, preferably having a smallest size below 60, preferably below 40 and more preferably below 30 nm and comprising metal compound nano-particles having an average particle size below 80, preferably below 60 and more preferably below 40 nm and wherein the cellulose and metal compound nano-particles are homogeneously mixed on nano-scale. Preferred hybrid nano-structured composites of the invention comprise as metal compound Zinc-chloride, -hydroxide, -oxide or -carbonate, Lanthanum-chloride, -hydroxide, -oxide or-carbonate or lithium-acetate.

The invention also relates to the use of the hybrid nano-structured composite of the invention for energy generation, in electronic devices, as a pigment and/or a pigment support, as a whitener or filler in food or personal care products, as anti-bacterial compound, in anti-bacterial clothing, in the flexible and optically transparent paper, foil, tape or cloth.

The nano-cellulose based hybrid nanomaterials have huge potential applications in food packaging, biopharmaceuticals, biomedical, cosmetics and electronics. The hybrid composite combines the properties of functional metallic materials with nano-cellulose and can be used as a base material or building block in several applications. For example, the hybrid composites are used in certain electronic devices as for instance in solar cells, flexible displays, ultracapacitors etc. where metals like Li, Mn, Ti, Zn, Ru and others are combined with nano-cellulose paper, foils, sheets, tapes etc. The hybrid nanocomposite containing cellulose- and zinc-oxide nanoparticles have excellent mechanical, UV barrier, and antibacterial properties.

In a particular aspect, the invention relates to the use of hybrid nano-structured composite wherein the metal compound is zinc-oxide or of cellulose nano-particles or of Zinc-oxide nano-particles or combinations thereof for replacing titanium-oxide as white pigment in food or personal care products or in paints, coatings and plastic articles.

## Experimental Methods

### Measurement of XRD crystal type

The cellulose products obtained in the experiments are characterised using XRD. XRD measurements according to the method described by: Z. Man, N. Muhammand, A. Sarwono, M.A. Bustam, M. Vignesh Kumar, S. Rafiq in J. Polym. Environ 19 (2011) 726-731: Preparation of cellulose nanocrystals using an Ionic liquid. The crystal type I or II was identified by peak positions, which are for type I on 2θ of 22.6° (the [200] reflection) and for type II on 2θ of 20° and 22° (the [110] and [020] reflection). Before XRD measurement the product samples were dried by vacuum drying at room temperature.

### Measurement of XRD crystallinity

The product crystallinity (mentioned in the above document as crystallinity index) was determined using Segal's formula: $CrI = (I_{002}-I_{am})/I_{002}$ wherein $I_{002}$ is the overall intensity of the peak at 2θ of 22.6° for type I or 22° for type II cellulose and $I_{am}$ is the intensity of the baseline at 2θ about 18°.

### Measurement of XRD crystal size

The cellulose crystal size was determined from the measured XRD using the Scherrer's equation:

$$\beta = \frac{0.9\lambda}{\tau \cos \theta}$$

wherein β is the crystallite sizes, λ is the wavelength of incident X-rays, τ is the full width at half maximum (FWHM) of the XRD peaks, θ is the diffraction angles corresponding to the planes.

### Measurement of cellulose product yield and cellulose hydrolyzation

Soluble (poly-)sugars were measured based on mass balance % of (poly-)sugars = 1 - $M^{prec}_{cel}/M^{in}_{cel}$ wherein $M^{prec}_{cel}$ is the weight of dry micro- or nano-cellulose obtained in the experiment and $M^{in}_{cel}$ is the weight of dry cellulose placed in the reactor. The term (poly-)sugars implies sugars and poly-sugars such as oligomer sugars. The drying of the obtained cellulose product is done according to the NREL lab procedure, convection oven drying for biomass is performed at 45° C for 24h - 48 h with regular (typically every 3 h) check of the weight until the dry biomass weight does not change more than 1 wt.% in one hour.

### Measurement of aspect ratio and crystal size

The aspect ratio and crystal size of the nano-cellulose can be analysed with a scanning electron microscope (SEM) or transmission electron microscope.

### Materials used

The cellulose base material in all the below described experiments is cotton linter Micro Crystalline Cellulose (MCC) ex-Sigma C6288. XRD characterization shows ± 80% of XRD-I type. $ZnCl_2$ and ZnO were also received from Sigma.

## DESCRIPTION OF EXPERIMENTS

### Example 1: NC-II/Zn(OH)$_2$

The first solvent was prepared by adding 0.5 g ZnO powder to 100 g aqueous solution of 60 wt.% $ZnCl_2$ in water, the mixture was kept under stirring (120 rpm/min) at room temperature overnight. Remaining unreacted ZnO solids were removed from the solution by filtration. The resulting 100 g solvent was mixed with 5 g of the cotton liner cellulose under stirring (480 rpm/min) and kept under stirring for 30 min at room temperature. The obtained cellulose type I crystals C-I were separated from the solution by filtration over a glass filter, washed 8 times with deionized water to remove $ZnCl_2$ and dried in vacuum at room temperature.

The highly crystalline cellulose type I crystals C-I were subsequently contacted with a second solvent. The second solvent is a molten metal salt solvent prepared by mixing 0.5 g ZnO powder (as proton scavenger) with 100 g aqueous solution of 70 to 75 wt% $ZnCl_2$ and kept under stirring at room temperature overnight. Remaining ZnO solids were removed from the solvent by filtration. 100 g of the second solvent was mixed with 5 g of the cellulose type I crystals C-I and stirred for 30 min at room temperature till the solution became clear.

225 g deionized water was added under stirring to the solution to decrease $ZnCl_2$ concentration to 20 wt.% to precipitate the cellulose from the second solution. The sample was kept under stirring for 20 min to allow the cellulose nanocrystals to precipitate. The precipitation causes the solution to gel. The gel precipitate is filtered. The cellulose to $ZnCl_2$ ratio in the gel precipitate is 1:10 based on dry solids weight.

The gel comprises high crystallinity high purity nano-cellulose having XRD type II (NC-II). XRD measurement of a dried sample of this precipitate shows that the cellulose XRD type I obtained in the first dissolution step is converted in the second step to nano cellulose XRD type II having an XRD crystallinity above 80% and less than 5 wt.% saccharide monomers or oligomers formed.

The gel precipitate is washed with water at $T_{room}$= 25°C for 2 times with a gel-to-fresh water volume ratio 1:8 until the product has a $ZnCl_2$ content of about 10 wt% (cellulose to $ZnCl_2$ 10:1 based on dry solids). The resulting product is referred to as product 1-A.

An amount of 100 gr of this product 1-A is then mixed at temperature T = 25°C with 0.1 gr NaOH producing a cloudy gel-like solution which after filtration results in a wet precipitate comprising a mixture of precipitated nano-cellulose type II crystals and precipitated zinc-hydroxide NC-II/$Zn(OH)_2$. The amount of $Zn(OH)_2$ in the hybrid composite can be optimized by using an excess of NaOH to ensure complete $Zn(OH)_2$ precipitation, followed by washing out residual NaOH. The solid co-precipitate NC-II/$Zn(OH)_2$ is separated and washed twice with water (precipitate to water volume ratio 1:8) to remove NaCl. The precipitate sample is thereafter dried at 80 °C.

### Example 2: NC-II/ZnO

In the above described preferred embodiment of Example 1 the nano-cellulose obtained have a high crystallinity and high purity and they degrade at higher temperature. It was found that high crystallinity high purity cellulose nanocrystals in dry state have excellent thermal stability and resist degradation at temperatures even above 200°C. This advantageous property can be used to thermally convert hybrid nano-structured composite of the invention at elevated temperatures. The precipitate sample NC-$Zn(OH)_2$ obtained in Example 1 is heated at 200°C to convert $Zn(OH)_2$ into ZnO. The final dry weight ratio cellulose to ZnO is 10: 0.75 as determined with DTA and XRF.

### Example 3: NC-II/LaCl$_3$

An amount of 100 g of the NC-II/$ZnCl_2$ product 1-A is contacted with a 1000 ml solution of $LaCl_3$ (comprising 2 gr $LaCl_3$ in 1000 ml water) whereby the La exchanges the Zn forming a hybrid composition NC-II/$LaCl_3$ with relative amounts cellulose: $LaCl_3$: $ZnCl_2$ 10:1:0.1.

### Example 4: NC-III La$_2$O$_3$

The product obtained in example B-1 is washed with NaOH to form $La(OH)_3$ which is converted by dehydration at T = 200°C under vacuum to $La_2O_3$ producing a NC-II/$La_2O_3$ hybrid composite.

### Example 5: Whiteness Test of : NC-III La$_2$O$_3$

Nano-cellulose NC was produced separately as described in example 1 and instead of adding Na-OH metal precipitation reactant, the NC nano-crystals were washed, filtered and dried. ZnO nanoparticles were commercially obtained. The whiteness of the hybrid nano-structured composite material NC-II/ZnO of Example 2 was compared with the pure cellulose nanocrystals, ZnO nano-particles and white paper. It appeared that the NC-II/ZnO of Example 2 was whiter than the constituents as shown in the Table below.

| sample | whiteness value |
|---|---|
| White Paper | 88 |
| NC | 90 |
| ZnO | 89 |
| NC-ZnO | 92 |

**Claims**

1. A method for preparing a hybrid nano-structured composite material comprising cellulose nano-particles and metal compound nano-particles comprising the steps of:

  a) Contacting virgin cellulose with a molten metal salt solvent $M_1$-S comprising metal ions $M_1$ and dissoluting the virgin cellulose,
  b) Adding a precipitation reactant B to convert at least part of the metal ions $M_1$ of the molten metal salt solvent $M_1$-S to metal compound nano-particles $M_1$_X or exchanging at least part of metal ions $M_1$ with metal ions $M_2$ by contacting with a solution of a salt comprising metal ions $M_2$ and precipitating metal compound $M_2$_X nano-particles directly or by adding a precipitation reactant B,
  c) Adding an anti-solvent and precipitating the cellulose nano-particles before, during or after step b),

d) Isolating the co-precipitated cellulose- and metal compound nano-particles to obtain the hybrid nano-structured composite material,

e) Optionally converting the metal compound in the hybrid nano-structured composite material into another metal compound.

2. The method of claim 1, wherein the molten metal salt is a $ZnCl_2$ hydrate, preferably $ZnCl_2.4H_2O$, which preferably is proton-free and preferably comprises a proton scavenger and wherein optionally the molten metal $M_1$ salt solvent comprises a metal cation $M_2$ other than the metal $M_1$ of the molten salt solvent, preferably comprising a $ZnCl_2$ molten metal salt solvent and a $M_2$ metal chloride, wherein the metal cation $M_2$ preferably is one or more chosen from the group of Li, Mn, Ti, Zn, Nd, Cd, Ag and Ru, most preferably $TiCl_2$, $MnCl_2$, $LiCl_2$ and wherein preferably the amount of $M_2$ metal is less than 20%, preferably less than 10 or 5 mole % of the amount of $M_1$.

3. The method of anyone of claims 1-2, comprising in step a) contacting virgin cellulose with a first solvent, **characterized in that** the first solvent is an aqueous solution comprising 40 - 65 wt.% $ZnCl_2$ in water, whereby the amorphous cellulose phase is preferentially dissolved over the crystalline cellulose phase, optionally separating the obtained crystalline cellulose having an XRD type I structure, and contacting the optionally separated crystalline cellulose having an XRD type I structure with a second solvent comprising a higher concentration than the first solvent of between 65 and 90 wt.% $ZnCl_2$ in water to produce delaminated cellulose having an XRD type II structure, wherein the second solvent and preferably also the first solvent is free of proton acid and preferably comprise a proton scavenger.

4. The method of anyone of claims 1 - 3, wherein the precipitation reactant B is a base anion X, preferably comprising a hydroxy, carbonate or carboxylate, wherein reactant B is preferably chosen from the group of NaOH, KOH, $KHCO_3$, a formiate or acetate salt, wherein precipitation reactant B forms a nano-particle precipitate Mi-Xwith metal $M_1$ and/or $M_2$-X with metal $M_2$ and wherein optionally the precipitation reactant B may comprise a metal ion $M_2$ other than metal $M_1$ or wherein the metal $M_1$ is exchanged with metal $M_2$ to form a precipitate of metal $M_2$-X, wherein the metal cation $M_2$ is preferably one or more chosen from the group of Li, Mn, Ti, Nd, Cd, Ag and Ru.

5. The method of anyone of claims 1 - 4, wherein the anti-solvent C is water, a ketone or alcohol, preferably water added in an amount to reduce the salt concentration of the molten salt, preferably the $ZnCl_2$ concentration, to between 10 and 30 wt.%, preferably between 15 and 25 wt.%.

6. The method of anyone of claims 1 - 5, wherein in step b) precipitation reactant B is added to convert at least part of the metal ions $M_1$ to a nano-particles of metal compound $M_1$-X followed by adding anti-solvent C in step c) to precipitate the cellulose nano-particles in the presence of the nano-particles of metal compound $M_1$X, optionally followed by one or more steps of filtration, washing and drying or preferably the method comprises step a) followed by step c) and then by step b) comprising adding anti-solvent C to the cellulose solution obtained in step a) forming a gel of precipitated cellulose nano-particles, optionally followed by separating the gel and washing to reduce the concentration of the metal salt of the molten salt solvent, followed by addition of precipitation reactant B in step b) to convert at least part of the metal ions $M_1$ to a nano-particles of metal compound $M_1$X in the presence of the cellulose nano-particles.

7. The method of claim 6, wherein the molten metal salt solvent is $ZnCl_2.4H_2O$ and the precipitation reactant B is a hydroxide base resulting in a hybrid composite of cellulose- and Zinc-hydroxide nanoparticles and wherein optionally the hybrid composite is treated to convert Zinc-hydroxide nanoparticles to Zinc-oxide nano-particles, preferably by drying at elevated temperatures between 70 and 350°C, preferably between 80 and 300°C, even more preferably between 80 and 280°C.

8. The method of anyone of claims 1 - 7, wherein step a) is followed by step c) wherein anti-solvent C is adding to the cellulose solution obtained in step a) forming a gel of precipitated cellulose nano-particles, optionally followed by separating the gel and washing to reduce the concentration of the metal salt of the molten salt solvent, followed by step b) wherein at least part of metal ions $M_1$ are exchanged with metal ions $M_2$ by contacting the solution with a solution of a salt comprising metal ions $M_2$, together with or followed addition of precipitation reactant B, forming precipitate of metal compound nano-particles $M_2$X.

9. The method of anyone of claims 1 - 8, comprising conversion step e) wherein the metal compound in the hybrid nano-structured composite material is converted into another metal compound, preferably by thermal decomposition, ion-exchange, reduction or oxidation.

10. The method of anyone of claims 1 - 9 wherein interlinking agents are added to link with the cellulose nanoparticles, which are preferably chosen from the groups of glycerol, citric acid, acetate or chitosan and preferably are organometal compounds of exchange metal $M_2$, preferably $M_2$ acetate or -citrate and which interlinking agents are preferably added in step b).

11. A hybrid nano-structured composite comprising cellulose nano-particles and metal compound nano-particles obtainable by the process of anyone of claims 1-10, preferably comprising 2-20 wt% metal compound relative to the total dry weight of the nano-structured composite, wherein the hybrid nano-structured composite preferably comprising cellulose nano-particles having X-Ray Diffraction (XRD) type II structure and preferably having an aspect ratio AR of at least 5, preferably at least 10, preferably having a smallest size below 60, preferably below 40 and more preferably below 30 nm and comprising metal compound nano-particles having an average particle size below 80, preferably below 60 and more preferably below 40 nm and wherein the cellulose and metal compound nano-particles are homogeneously mixed on nano-scale.

12. The hybrid nano-structured composite of claim 11 wherein the metal compound is Zinc-chloride, -hydroxide, -oxide or -carbonate, Lanthanum-chloride, -hydroxide, -oxide or - carbonate, lithium-acetate.

13. Use of the hybrid nano-structured composite of claims 11 or 12 for energy generation, in electronic devices, as a pigment and/or a pigment support, as a whitener or filler in food or personal care products, as anti-bacterial compound, in anti-bacterial clothing, in the flexible and optically transparent paper, foil, tape or cloth.

14. Use of the hybrid nano-structured composite of claims 11 or 12 wherein the metal compound is zinc-oxide or of cellulose nano-particles or of Zinc-oxide nano-particles or combinations thereof for replacing titanium-oxide as white pigment in food or personal care products or in paints, coatings and plastic articles.

Fig 1.  Example A

Fig 2.  Example A1

Fig 3.  Example B

M₁-S → [ C_s ] → NC_d - M₁-S → H₂O → [ ] → M₁-S

NC_s - M₁S

Concentrate and re-use

KHCO3 → [ ]

**NC_s - M₁HCO3**  (+ KS)

Fig 4.  Example B1

ZnCl₂.4H₂O → [ C_s ] → NC_d–ZnCl₂ → H₂O → [ ] → ZnCl₂

NC_s - ZnCl₂

Concentrate and re-use

KHCO₃ → [ ]

**NC_s – Zn(HCO₃)₂**  (+ KCl)

EP 4 053 206 A1

Fig 5. Example C

Fig 6. Example C1

13

Europäisches
Patentamt

European
Patent Office

Office européen
des brevets

# EUROPEAN SEARCH REPORT

Application Number

EP 21 16 0301

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MA JINXIA ET AL: "Preparation of ZnO-cellulose nanocomposites by different cellulose solution systems with a colloid mill", CELLULOSE, SPRINGER NETHERLANDS, NETHERLANDS, vol. 23, no. 6, 8 October 2016 (2016-10-08), pages 3703-3715, XP036104397, ISSN: 0969-0239, DOI: 10.1007/S10570-016-1081-0 [retrieved on 2016-10-08] * abstract; page 3705, col.1, para.2-page 3706, col.2, para.1; fig.1; Conclusions * ----- | 1-14 | INV. C08L1/02 C08K3/22 C08K3/105 C08K3/11 |
| X | FAROOQ AMJAD ET AL: "Cellulose from sources to nanocellulose and an overview of synthesis and properties of nanocellulose/zinc oxide nanocomposite materials", INTERNATIONAL JOURNAL OF BIOLOGICAL MACROMOLECULES, vol. 154, 1 July 2020 (2020-07-01), pages 1050-1073, XP055832401, NL ISSN: 0141-8130, DOI: 10.1016/j.ijbiomac.2020.03.163 Retrieved from the Internet: URL:https://www.sciencedirect.com/science/article/pii/S0141813020303585/pdfft?md5=9de9f25f8a696da56bd5ec986d73b013&pid=1-s2.0-S0141813020303585-main.pdf> * abstract; paragraph 3.4 * ----- | 11-14 | |
| | -/-- | | TECHNICAL FIELDS SEARCHED (IPC) C08L C08K |

The present search report has been drawn up for all claims

| Place of search | Date of completion of the search | Examiner |
|---|---|---|
| Munich | 17 August 2021 | Pellegrini, Paolo |

Europäisches Patentamt

European Patent Office

Office européen des brevets

**EUROPEAN SEARCH REPORT**

Application Number

EP 21 16 0301

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RUSZALA M. J. A. ET AL: "Low Carbon Footprint TiO2 Substitutes in Paint: A Review", INTERNATIONAL JOURNAL OF CHEMICAL ENGINEERING AND APPLICATIONS, vol. 6, no. 5, 1 October 2015 (2015-10-01), pages 331-340, XP055832412, SG ISSN: 2010-0221, DOI: 10.7763/IJCEA.2015.V6.505 Retrieved from the Internet: URL:http://dx.doi.org/10.7763/IJCEA.2015.V6.505> * paragraph V.A.1 * | 14 | |

TECHNICAL FIELDS SEARCHED (IPC)

The present search report has been drawn up for all claims

| Place of search | Date of completion of the search | Examiner |
|---|---|---|
| Munich | 17 August 2021 | Pellegrini, Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017055407 A
- WO 2020212616 A
- WO 2019229030 A1

**Non-patent literature cited in the description**

- **DELGADO-AGUILAR et al.** *in BioResources,* 2015, vol. 10 (3), 5345-5355
- *International Journal of Biological Macromolecules,* 2020, vol. 154, 1050-1073
- **Z. MAN ; N. MUHAMMAND ; A. SARWONO ; M.A. BUSTAM ; M. VIGNESH KUMAR ; S. RAFIQ.** *J. Polym. Environ,* 2011, vol. 19, 726-731